(19) Europäisches Patentamt
European Patent Office
Office européen des brevets

(11) EP 3 650 434 A1

(12) EUROPEAN PATENT APPLICATION

(43) Date of publication:
13.05.2020 Bulletin 2020/20

(51) Int Cl.:
*C07C 45/46* (2006.01) *C07C 49/84* (2006.01)

(21) Application number: 18306472.4

(22) Date of filing: 09.11.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Arkema France**
**92700 Colombes (FR)**

(72) Inventors:
- **JOUANNEAU, Julien**
**KING OF PRUSSIA, PA Pennsylvania 19406-0936 (US)**
- **LE, Guillaume**
**27470 SERQUIGNY (FR)**
- **HERBLOT, Martin**
**27470 SERQUIGNY (FR)**

# (54) METHOD FOR MANUFACTURING 1,4-BIS (4-PHENOXYBENZOYLBENZENE) USING SUBSTANTIALLY NON-HYDROLYZED TEREPHTHALOYL CHLORIDE

(57) A method for manufacturing 1,4-bis(4-phenoxybenzoyl)benzene, comprising:
- providing a solvent, a Lewis acid, a first reactant and a second reactant,
wherein the first reactant and the second reactant are respectively terephthaloyl chloride and diphenyl ether, or reversely;
wherein the terephthaloyl chloride is of a purity grade such that, 10 minutes after introducing it at a reference concentration of 6.5 wt.% into ortho-dichlorobenzene containing less than 100 ppm by weight of water, at a temperature of 20°C, a solution is obtained having a turbidity of less than 500 NTU;
- mixing the first reactant in the solvent to make a starting mixture; and,
- adding the second reactant to the starting mixture;
wherein the Lewis acid is mixed to the starting mixture before adding the second reactant and/or with the second reactant.

A method for manufacturing a polyether ketone ketone polymer starting from 1,4-bis(4-phenoxybenzoyl)benzene manufactured by the above method.

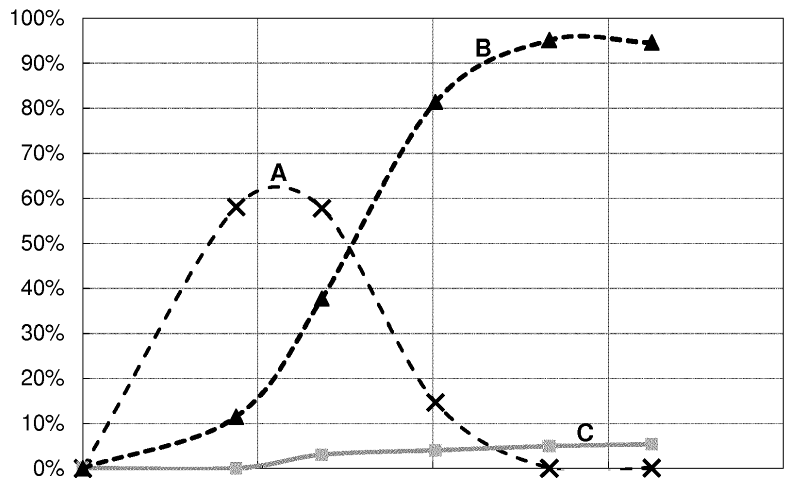


Fig. 2

EP 3 650 434 A1

## Description

### TECHNICAL FIELD

The present application relates to a method for manufacturing 1,4-bis(4-phenoxybenzoyl)benzene, as well as a method for manufacturing polyaryl etherketone polymers, in particular polyether ketone ketone polymers, starting from said 1,4-bis(4-phenoxybenzoyl)benzene.

### TECHNICAL BACKGROUND

Polyether ketone ketone (abbreviated name: PEKK) polymers have a number of properties which make them useful for applications involving exposure to high temperature or to high mechanical or chemical stress. They are for instance useful in the aerospace industry, in off-shore drilling and in medical devices.

One known route for manufacturing polyether ketone ketone polymers relies on the use of 1,4-bis(4-phenoxybenzoyl)benzene as a starting material.

1,4-bis(4-phenoxybenzoyl)benzene can be prepared by reacting terephthaloyl chloride and diphenyl ether in the presence of a Lewis acid such as aluminum trichloride.

In document US 4,816,556 (example 2), 1,4-bis(4-phenoxybenzoyl)benzene is prepared by dissolving terephthaloyl chloride and diphenyl ether in ortho-dichlorobenzene and adding aluminum chloride. Thereafter, cold methanol is added so as to produce a slurry which is filtered, reslurried in methanol and filtered again.

In document US 4,826,947 (example 2), 1,4-bis(4-phenoxybenzoyl)benzene is prepared by providing a mixture of methylene chloride, methylsulfone and aluminum trichloride, adding diphenyl ether and thereafter terephthaloyl chloride. The reaction mixture is then poured into cold methanol so as to make a slurry which is then filtered.

Document WO 95/23821 (example 11) discloses providing aluminum chloride in ortho-dichlorobenzene and then adding terephthaloyl chloride and diphenyl ether. Thereafter, the reaction mixture is allowed to warm up to room temperature, stirred, and poured into a methanol concentrated HCl solution. A precipitate is formed which is subsequently filtered off.

There is still a need for new methods for manufacturing 1,4-bis(4-phenoxybenzoyl)benzene with a high purity and a high yield, which can be implemented at the industrial scale in an economically realistic manner.

### SUMMARY

It is a first object of the invention to provide a method for manufacturing 1,4-bis(4-phenoxybenzoyl)benzene, comprising:

- providing a solvent, a Lewis acid, a first reactant and a second reactant,
  wherein the first reactant and the second reactant are respectively terephthaloyl chloride and diphenyl ether, or reversely;
  wherein the terephthaloyl chloride is of a purity grade such that, 10 minutes after introducing it at a reference concentration of 6.5 wt.% into ortho-dichlorobenzene containing less than 100 ppm by weight of water, at a temperature of 20°C, a solution is obtained having a turbidity of less than 500 NTU;
- mixing the first reactant in the solvent to make a starting mixture; and,
- adding the second reactant to the starting mixture;
  wherein the Lewis acid is mixed, at least partly, to the starting mixture before adding the second reactant to the starting mixture, and/or
  wherein the Lewis acid is mixed, at least partly, with the second reactant and added together to the starting mixture;

so as to obtain a product mixture comprising a 1,4-bis(4-phenoxybenzoyl)benzene-Lewis acid complex.

In some embodiments, the terephthaloyl chloride is of a purity grade such that:

- 10 minutes after introducing it at a reference concentration of 6.5 wt.% into ortho-dichlorobenzene containing less than 100 ppm by weight of water, at a temperature of 20°C, a solution is obtained having a turbidity of less than 200 NTU, preferably of less than 100 NTU, more preferably of less than 50 NTU, and most preferably of less than 10 NTU;
- preferably, 10 hours after introducing it at a reference concentration of 6.5 wt.% into ortho-dichlorobenzene containing less than 100 ppm by weight of water, at a temperature of 20°C, a solution is obtained having a turbidity of less than 500 NTU, preferably of less than 200 NTU, more preferably of less than 100 NTU, even more preferably of less than 50 NTU and most preferably of less than 10 NTU; and
- more preferably, 24 hours after introducing it at a reference concentration of 6.5 wt.% into ortho-dichlorobenzene

containing less than 100 ppm by weight of water, at a temperature of 20°C, a solution is obtained having a turbidity of less than 500 NTU, preferably of less than 200 NTU, more preferably of less than 100 NTU, even more preferably of less than 50 NTU and most preferably of less than 10 NTU.

In some embodiments, the terephthaloyl chloride is kept in a sealed container without contact with ambient air before use.

In some embodiments, the diphenyl ether and solvent, in combination, contain less than 500 ppm by weight of water, advantageously less than 250 ppm by weight of water, preferably less than 150 ppm by weight of water, more preferably less than 100 ppm by weight of water, and most preferably less than 50 ppm by weight of water.

In some embodiments, the diphenyl ether and solvent, in combination, contain from 1 to 250 ppm by weight of water, preferably from 2 to 200 ppm by weight of water, more preferably from 3 to 150 ppm by weight of water, even more preferably from 4 to 100 ppm by weight of water and most preferably from 5 to 50 ppm by weight of water.

In some embodiments, the method comprises a step of drying the solvent and/or the diphenyl ether before use, preferably by distillation or by contacting with a molecular sieve or with a dehydrating agent.

In some embodiments, the 1,4-bis(4-phenoxybenzoyl)benzene-Lewis acid complex is dissolved in the solvent at a 1,4-bis(4-phenoxybenzoyl)benzene weight concentration in the solvent which is higher than the saturation limit of the 1,4-bis(4-phenoxybenzoyl)benzene-Lewis acid complex during at least part of the reaction of the terephthaloyl chloride with the diphenyl ether.

In some embodiments, the 1,4-bis(4-phenoxybenzoyl)benzene-Lewis acid complex is dissolved in the solvent at a 1,4-bis(4-phenoxybenzoyl)benzene weight concentration which is higher by at least 5 %, preferably by at least 10 %, more preferably by at least 20 %, than the saturation limit of the 1,4-bis(4-phenoxybenzoyl)benzene-Lewis acid complex, during part of the reaction of the terephthaloyl chloride with the diphenyl ether.

In some embodiments, the 1,4-bis(4-phenoxybenzoyl)benzene-Lewis acid complex is dissolved in the solvent at a 1,4-bis(4-phenoxybenzoyl)benzene weight concentration of more than 5 wt.%, preferably more than 10 wt.%, more preferably more than 15 wt.%, and most preferably more than 30 wt.%, during part of the reaction of the terephthaloyl chloride with the diphenyl ether.

In some embodiments, the Lewis acid is aluminum trichloride.

In some embodiments, the solvent is different from diphenyl ether, terephthaloyl chloride or the Lewis acid. The solvent may advantageously be ortho-dichlorobenzene.

On the contrary, in some other embodiments, the solvent is either diphenyl ether, terephthaloyl chloride or the Lewis acid. The solvent may advantageously be diphenyl ether.

In some embodiments, the second reactant is terephthaloyl chloride. The Lewis acid is mixed with terephthaloyl chloride and added together to the starting mixture.

In some embodiments, the first reactant is terephthaloyl chloride. The Lewis acid is mixed with the starting mixture before adding the diphenyl ether to the starting mixture.

In some embodiments, the method of the invention comprises one or more steps for purifying 1,4-bis(4-phenoxybenzoyl)benzene-Lewis acid complex from the product mixture.

The invention also relates to a method of making a polyether ketone ketone polymer, comprising:

- manufacturing 1,4-bis(4-phenoxybenzoyl)benzene according to the method of anyone of the disclosed embodiments;
- reacting said 1,4-bis(4-phenoxybenzoyl)benzene with at least one difunctional aromatic acyl chloride.

Independently from the above, the invention additionally provides the following items:

Item 1. A method for manufacturing 1,4-bis(4-phenoxybenzoyl)benzene, comprising:

- providing a solvent, a Lewis acid, a first reactant and a second reactant,
  wherein the first reactant and the second reactant are respectively terephthaloyl chloride and diphenyl ether, or reversely, and
  wherein the diphenyl ether and solvent, in combination, contain less than 500 ppm by weight of water;
- mixing the first reactant in the solvent to make a starting mixture; and,
- adding the second reactant to the starting mixture;
  wherein the Lewis acid is mixed, at least partly, to the starting mixture before adding the second reactant to the starting mixture, and/or
  wherein the Lewis acid is mixed, at least partly, with the second reactant and added together to the starting mixture;

so as to obtain a product mixture comprising a 1,4-bis(4-phenoxybenzoyl)benzene-Lewis acid complex.

Item 2. The method of item 1, wherein the diphenyl ether and solvent, in combination, contain less than 250 ppm by weight of water, preferably less than 150 ppm by weight of water, more preferably less than 100 ppm by weight of water, and most preferably less than 50 ppm by weight of water.

Item 3. The method of item 1 or item 2, wherein the terephthaloyl chloride is of a purity grade such that, 10 minutes after introducing it at a reference concentration of 6.5 wt.% into ortho-dichlorobenzene containing less than 100 ppm by weight of water, at a temperature of 20°C, a solution is obtained having a turbidity of less than 500 NTU.

Item 4. The method of any of items 1 to 3, wherein the terephthaloyl chloride is of a purity grade such that:

- 10 minutes after introducing it at a reference concentration of 6.5 wt.% into ortho-dichlorobenzene containing less than 100 ppm by weight of water, at a temperature of 20°C, a solution is obtained having a turbidity of less than 200 NTU, preferably of less than 100 NTU, more preferably of less than 50 NTU, and most preferably of less than 10 NTU;
- preferably, 10 hours after introducing it at a reference concentration of 6.5 wt.% into ortho-dichlorobenzene containing less than 100 ppm by weight of water, at a temperature of 20°C, a solution is obtained having a turbidity of less than 500 NTU, preferably of less than 200 NTU, more preferably of less than 100 NTU, even more preferably of less than 50 NTU and most preferably of less than 10 NTU; and
- more preferably, 24 hours after introducing it at a reference concentration of 6.5 wt.% into ortho-dichlorobenzene containing less than 100 ppm by weight of water, at a temperature of 20°C, a solution is obtained having a turbidity of less than 500 NTU, preferably of less than 200 NTU, more preferably of less than 100 NTU, even more preferably of less than 50 NTU and most preferably of less than 10 NTU.

Item 5. The method of any of items 1 to 4, wherein the diphenyl ether and solvent, in combination, contain from 1 to 250 ppm of water, preferably from 2 to 200 ppm by weight of water, more preferably from 3 to 150 ppm by weight of water, even more preferably from 4 to 100 ppm by weight of water and most preferably from 5 to 50 ppm by weight of water.

Item 6. The method of any of items 1 to 5, comprising a step of drying the solvent and/or a step of drying the diphenyl ether before use, preferably by distillation or by contacting with a molecular sieve or with a dehydrating agent.

Item 7. The method of any of items 1 to 6, wherein the terephthaloyl chloride is kept in a sealed container without contact with ambient before use.

Item 8. The method of any of items 1 to 7, wherein the 1,4-bis(4-phenoxybenzoyl)benzene-Lewis acid complex is dissolved in the solvent at a 1,4-bis(4-phenoxybenzoyl)benzene weight concentration in the solvent which is higher than the saturation limit of the 1,4-bis(4-phenoxybenzoyl)benzene-Lewis acid complex during at least part of the reaction of the terephthaloyl chloride with the diphenyl ether.

Item 9. The method of any of items 1 to 8, wherein the 1,4-bis(4-phenoxybenzoyl)benzene-Lewis acid complex is dissolved in the solvent at a 1,4-bis(4-phenoxybenzoyl)benzene weight concentration which is higher by at least 5 %, preferably by at least 10 %, more preferably by at least 20 %, than the saturation limit of the 1,4-bis(4-phenoxybenzoyl)benzene-Lewis acid complex, during part of the reaction of the terephthaloyl chloride with the diphenyl ether.

Item 10. The method of any of items 1 to 9, wherein the 1,4-bis(4-phenoxybenzoyl)benzene-Lewis acid complex is dissolved in the solvent at a 1,4-bis(4-phenoxybenzoyl)benzene weight concentration of more than 5 wt.%, preferably more than 10 wt.%, more preferably more than 15 wt.%, and most preferably more than 30 wt.%, during part of the reaction of the terephthaloyl chloride with the diphenyl ether.

Item 11. The method of any one of any of items 1 to 10, wherein the 1,4-bis(4-phenoxybenzoyl)benzene-Lewis acid complex is dissolved in the solvent during at least 75 %, preferably at least 90 %, more preferably at least 95 % of the duration of the reaction of the terephthaloyl chloride with the diphenyl ether, and most preferably during the totality of the reaction of the terephthaloyl chloride with the diphenyl ether.

Item 12. The method of any one of any of items 1 to 11, wherein the 1,4-bis(4-phenoxybenzoyl)benzene-Lewis acid complex is dissolved in the solvent when an amount of 1,4-bis(4-phenoxybenzoyl)benzene of 75 mol.%, relative to the initial amount of terephthaloyl chloride, is present in the starting mixture; preferably the 1,4-bis(4-phenoxybenzoyl)benzene-Lewis acid complex is dissolved in the solvent when an amount of 1,4-bis(4-phenoxybenzoyl)benzene of 80 mol.%, relative to the initial amount of terephthaloyl chloride, is present in the starting mixture; more preferably the 1,4-bis(4-phenoxybenzoyl)benzene-Lewis acid complex is dissolved in the solvent when an amount of 1,4-bis(4-phenoxybenzoyl)benzene of 85 mol.%, relative to the initial amount of terephthaloyl chloride, is present in the starting mixture; and most preferably, the 1,4-bis(4-phenoxybenzoyl)benzene-Lewis acid complex is dissolved in the solvent when an amount of 1,4-bis(4-phenoxybenzoyl)benzene of 90 mol.%, relative to the initial amount of terephthaloyl chloride, is present in the starting mixture.

Item 13. The method of any of items 1 to 12, wherein the Lewis acid is aluminum trichloride.

Item 14. The method of any one of items 1 to 13, wherein the Lewis acid, the first reactant or the second reactant

plays the role of the solvent.

Item 15. The method of any of items 1 to 13, wherein the solvent is ortho-dichlorobenzene.

Item 16. The method of any one of claims 1 to 15, wherein the second reactant is terephthaloyl chloride and, wherein the Lewis acid is mixed with terephthaloyl chloride and added together to the starting mixture.

Item 17. The method of any one of items 1 to 15, wherein the first reactant is terephthaloyl chloride and, wherein the Lewis acid is mixed with the starting mixture before adding the diphenyl ether to the starting mixture.

Item 18. The method of any of items 1 to 17, comprising the additional steps of:

- mixing the product mixture with a protic solvent so as to provide a product slurry;
- separating 1,4-bis(4-phenoxybenzoyl)benzene from the product slurry, preferably by filtration and optionally washing.

Item 19. A method of making a polyether ketone ketone polymer, comprising:

- manufacturing 1,4-bis(4-phenoxybenzoyl)benzene according to the method of any of items 1 to 18;
- reacting said 1,4-bis(4-phenoxybenzoyl)benzene with at least one difunctional aromatic acyl chloride.

The present disclosure provides a method for manufacturing 1,4-bis(4-phenoxybenzoyl)benzene with a high purity and a high yield. This method can be implemented at the industrial scale.

In a first aspect, it has been found that the quality of the terephthaloyl chloride used as a starting material is critical for achieving high purity and high yield in the manufacture of 1,4-bis(4-phenoxybenzoyl)benzene. In particular, one acyl chloride group or both acyl chloride groups in terephthaloyl chloride may react with water potentially present in the material's environment. The presence of hydrolyzed forms of terephthaloyl chloride is undesirable for two reasons: first, the hydrolyzed portion of terephthaloyl chloride will not lead to the production of 1,4-bis(4-phenoxybenzoyl)benzene-Lewis acid complex; second, the hydrolyzed portion of terephthaloyl chloride tends to be insoluble in the solvent used for the reaction.

It is desirable to maintain the 1,4-bis(4-phenoxybenzoyl)benzene-Lewis acid complex which is produced during the reaction as a supersaturated solution during at least part of the reaction, in order to achieve a high yield of 1,4-bis(4-phenoxybenzoyl)benzene and a low level of by-product impurities such as 4-(4-phenoxybenzoyl)benzoyl chloride and its corresponding carboxylic acid and ester forms. In this context, and without wishing to be bound by any theory, it is believed that the hydrolyzed portion of terephthaloyl chloride in the starting material may act as a nucleating agent for the precipitation of the 1,4-bis(4-phenoxybenzoyl)benzene-Lewis acid complex and thus prevent said complex from being maintained as a supersaturated solution during the reaction.

In a second aspect, it has been found that the presence of even relatively low amounts of water during the reaction of terephthaloyl chloride and diphenyl ether may lead to a relatively large decrease in reaction yield and / or in the purity of final product. The presence of water may indeed lead to the hydrolysis of the terephthaloyl chloride, which is undesirable for the reasons already set forth above. In addition, water droplets may also act as a nucleating agent of their own.

By maintaining the amount of water in the solvent and diphenyl ether below a desired threshold, the yield of 1,4-bis(4-phenoxybenzoyl)benzene may be increased, and the level of by-product impurities such as 4-(4-phenoxybenzoyl)benzoyl chloride and its corresponding carboxylic acid and ester forms may remain low.

In a third aspect, the two-step addition of the two reactants, namely terephthaloyl chloride and diphenyl ether, enable to obtain 1,4-bis(4-phenoxybenzoyl)benzene-Lewis acid complex, and therefore 1,4-bis(4-phenoxybenzoyl)benzene, with a high purity and a high yield, in an efficient way. In particular, it enables to avoid any prior contacting time between the two reactants as in the methods of the prior art, in which the two reactants have to be first mixed together for several hours and heated before adding the second reactant. It also enables to reduce the production of unwanted by-products such as 4-(4-phenoxybenzoyl)benzoic acid or 4-(4-phenoxybenzoyl)benzoic acid ester.

## BRIEF DESCRIPTION OF THE DRAWINGS

**Fig.1** shows the saturation limit of the 1,4-bis(4-phenoxybenzoyl)benzene-Lewis acid complex in ortho-dichlorobenzene, as a function of temperature. Temperature in °C is provided on the X axis. The weight concentration of 1,4-bis(4-phenoxybenzoyl)benzene at saturation is provided in the Y axis. The Lewis acid-to-1,4-bis(4-phenoxybenzoyl)benzene molar ratio is 4.05. The Lewis acid is aluminum trichloride.

**Fig.2** schematically shows the evolution of the concentration of 4-(4-phenoxybenzoyl)benzoyl chloride (as defined below) (A), 1,4-bis(4-phenoxybenzoyl)benzene (B) and xanthydrol moiety-containing molecules (C), as a function

of the progression of the reaction. The concentrations are provided in mol.% relative to the initial total amount of terephthaloyl chloride. The progression of the reaction is indicated in arbitrary units on the X-axis.

DESCRIPTION OF EMBODIMENTS

The embodiments of the invention will now be described in more detail without limitation in the following description.

1,4-bis(4-phenoxybenzoyl)benzene (abbreviated name: EKKE) is the compound of formula I:

(I) 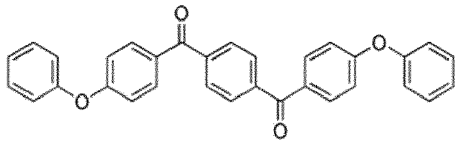

It may be made by reacting together terephthaloyl chloride and diphenyl ether, hereafter called "first reactant" and "second reactant". In some embodiment the first reactant is terephthaloyl chloride and the second reactant is diphenyl ether. On the contrary, in other embodiments, the first reactant is diphenyl ether and the second reactant is terephthaloyl chloride.

Terephthaloyl chloride is of formula II:

(II) 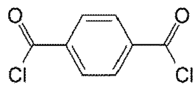

Diphenyl ether of formula III:

(III) 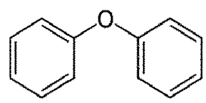

The reaction is carried out in a solvent, and in the presence of a Lewis acid, acting as a Friedel-Crafts catalyst. This is hereafter called *"the reaction step".*

The reaction results in the production of the compound of formula I which is predominantly in the form of a complex with the Lewis acid.

It is believed that the reaction comprises two stages. In the first stage, one molecule of formula II reacts with one molecule of formula III to form the following intermediate of formula IV (4-(4-phenoxybenzoyl)benzoyl chloride) which is called an *"active intermediate":*

(IV) 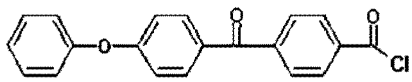

Then one molecule of the active intermediate of formula IV reacts with another molecule of formula III to form the desired product of formula I.

During the reaction, the following 4-(4-phenoxybenzoyl)benzoic acid of formula IVa can also be produced to some extent (notably from the active intermediate of formula IV):

(IVa) 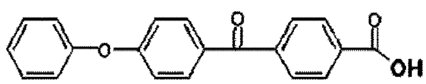

The corresponding 4-(4-phenoxybenzoyl)benzoic acid ester can be formed either directly from the acyl chloride of formula IV or from the carboxylic acid of formula IVa. The acid form and/or the ester form of the intermediate can be

formed during the reaction but they can also primarily be formed from the remaining active intermediate during subsequent workup (such as when the product mixture is mixed with a protic solvent, as described below).

The 4-(4-phenoxybenzoyl)benzoic acid and 4-(4-phenoxybenzoyl)benzoic acid ester are inactive and therefore remain as impurities in the product mixture.

The other main impurities produced by the reaction are xanthydrol moiety-containing molecules of formula (V):

(V) 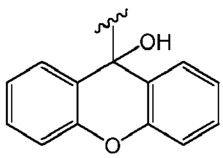

In some embodiments, one of the two reactants or the Lewis acid can play the role of the solvent.

On the opposite, in other embodiments, the solvent can be a separate solvent than the diphenyl ether, the terephthaloyl chloride, or the Lewis acid. In these embodiments, the solvent is preferably a non-protic solvent, which can in particular be selected from methylene chloride, carbon disulfide, ortho-dichlorobenzene, meta-dichlorobenzene, para-dichlorobenzene, 1,2,4-trichlorobenzene, 1,2,3-trichlorobenzene, ortho-difluorobenzene, 1,2-dichloroethane, 1,1-dichloroethane, 1,1,2,2-tetrachloroethane, tetrachloroethylene, dichloromethane, nitrobenzene and mixtures thereof.

Ortho-dichlorobenzene is a preferred solvent.

According to one aspect of the invention, the compound of formula II used as a starting material is provided with a high purity grade. Hydrolyzed forms of the compound of formula II are difficult to analyze and quantify in the starting material. It has been found that one practical method to ensure that the compound of formula II has the requisite purity is to dissolve it in a solvent, and to analyze its turbidity. It has been found that a high turbidity value in this test (which is indicative of a significant amount of insoluble contaminants within the starting material) results in a poor yield and a high amount of undesirable by-products in the manufacture of the compound of formula I.

Accordingly, the compound of formula II is preferably of a purity grade such that, 10 minutes after introducing it at a reference concentration of 6.5 wt.% in the solvent, at a temperature of 20°C, a solution is obtained having a turbidity of less than 500 NTU. Turbidity values are provided relative to a sample of solvent without compound of formula II.

In some embodiments, the solvent used in the turbidity test can be the same solvent as the reaction solvent. On the contrary, in other embodiments, the solvent used in the turbidity test can be a different solvent than the reaction solvent.

Preferably, the turbidity is less than 200 NTU, more preferably less than 100 NTU, even more preferably less than 50 NTU and most preferably less than 10 NTU.

A more stringent test consists in waiting for 10 hours, or even for 24 hours, between the introduction of the compound of formula II into the solvent and the turbidity measurement. Preferably, turbidity values of less than 500 NTU, preferably less than 200 NTU, more preferably less than 100 NTU, even more preferably less than 50 NTU and most preferably less than 10 NTU are obtained in this more stringent test.

In some variations, a second turbidity value may be obtained with a sample of solvent of so-called anhydrous grade; which is stored on a molecular sieve. Preferably, the anhydrous grade of solvent is characterized by a water weight content of less than 50 ppm, preferably of less than 30 ppm. In embodiments, the second, anhydrous turbidity value may be less than 500 NTU, preferably less than 200 NTU, more preferably less than 100 NTU, even more preferably less than 50 NTU and most preferably less than 10 NTU.

In other variations, the turbidity values indicated herein are obtained using a sample of the batch of solvent which is used for the reaction itself. This is particularly advantageous for the turbidity measurements conducted 10 hours or 24 hours after the introduction of the compound into the solvent, since the measurement is then representative of the actual quality of the compound in the reaction conditions, depending also on the quality of the solvent.

In a particular embodiment, the solvent used for the turbidity test is ortho-dichlorobenzene containing less than 100 ppm by weight of water. The turbidity may be assessed by introducing 1580 mg of terephthaloyl chloride into 22.6 g of ortho-dichlorobenzene containing less than 100 ppm by weight of water in a 50 mL flask, and by agitating the mixture with a magnetic stirrer, under nitrogen atmosphere. After 10 minutes, the turbidity of the mixture may be measured on a 20 mL sample with a Hach Lange Turbidimeter, using the same solvent as a blank sample.

The ortho-dichlorobenzene used for the turbidity test can have a content by weight of water of from 50 to 75 ppm; or from 75 to 100 ppm. The ortho-dichlorobenzene used for the turbidity test can also have a content by weight of water less than 50ppm, in particular from 1 to 5 ppm; or from 5 to 10 ppm; or from 10 to 20 ppm; or from 20 to 30 ppm; or from 30 to 40 ppm; or from 40 to 50 ppm.

A number of steps can be taken in order to ensure that the compound of formula II is of a satisfactory purity grade. In particular, the compound should substantially not be in contact with water at any time before the reaction. It can thus be advantageous to keep this material in a sealed container without contact with ambient air. Keeping the

material in a nitrogen atmosphere may in particular be useful.

As a sealed container, use may in particular be made of a container having walls and a lid having a moisture vapor transmission rate of not more than 0.1 $g/m^2$.24h at a relative humidity of 90% and a temperature of 37.8°C.

The container walls may be for instance made of polyethylene, such as high-density polyethylene.

The container walls may preferably have a thickness of at least 0.5 mm, more preferably at least 1 mm.

According to another aspect of the invention, the solvent and the compound of formula III used for the reaction have a low water content.

Accordingly, the solvent used for the reaction preferably contains less than 500 ppm by weight of water, advantageously less than 250 ppm by weight of water, preferably less than 150 ppm by weight of water, more preferably less than 100 ppm by weight of water, and most preferably less than 50 ppm by weight of water. In preferred variations, the above weight ranges also apply in a similar manner to the compound of formula III and the solvent in combination.

Possible weight ranges of water content in the solvent used for the reaction are: from 1 to 5 ppm; or from 5 to 10 ppm; or from 10 to 20 ppm; or from 20 to 30 ppm; or from 30 to 40 ppm; or from 40 to 50 ppm; or from 50 to 75 ppm; or from 75 to 100 ppm; or from 100 to 150 ppm; or from 150 to 200 ppm; or from 200 to 250 ppm; or from 250 to 300 ppm; or from 300 to 350 ppm; or from 350 to 400 ppm; or from 400 to 500 ppm. Ranges of from 1 to 250 ppm, or from 2 to 200 ppm, or from 3 to 150 ppm, or from 4 to 100 ppm, or from 5 to 50 ppm are particularly preferred. In preferred variations, the above weight ranges also apply in a similar manner to the compound of formula III and the solvent in combination.

A number of steps can be taken in order to ensure that the compound of formula III and/or the solvent do not contain an excessive amount of water. In particular, it is desirable to keep these materials in sealed containers without contact with ambient air. Keeping the materials in a dry nitrogen atmosphere may in particular be useful.

In some variations, the method of embodiments of the invention comprises a preliminary step of drying, *i.e.,* reducing the water content, of the solvent and/or the compound of formula III, before use. This step may be performed, *e.g.,* by distillation or by contacting with a molecular sieve or with a dehydrating agent.

By way of example, the weight proportion of water in the solvent, or in the solvent combined with the compound of formula III, may be reduced owing to the drying step from an initial value of 50 to 300 ppm to a final value of less than 100 ppm, preferably less than 50 ppm, more preferably less than 30 ppm.

The Lewis acid used in the method of embodiments of the invention is preferably of a purity grade such that it comprises less than 0.1 wt.% insoluble matter, and more preferably less than 0.05 wt.% insoluble matter, as measured by gravimetry, when introduced into the solvent at a concentration of 5 wt.% and substantially dissolved under agitation.

The reaction between the compounds of formulas II and III to make the compound of formula I may be performed in a reactor. The reactor can be for instance a glass reactor, a glass-lined reactor or a stainless-steel reactor.

Preferably, the reaction may be performed under a substantially water-free atmosphere, such as a dry nitrogen atmosphere.

According to some variations, the materials introduced into the reactor in the method of embodiments of the invention consist essentially, or consist, of the compounds of formulas II and III, the solvent and the Lewis acid.

According to other variations, the materials introduced into the reactor in the method of embodiments of the invention additionally comprise one or more additives for increasing the solubility of Lewis acid complexes. These additives may notably be selected from Lewis bases such as diphenylsulfone, carboxylic acids, ethers and inorganic salts such as LiCl, NaCl, KCl, $CaCl_2$ and $MgCl_2$, as well as mixtures thereof.

According to the invention, a starting mixture comprising the first reactant in a solvent, preferably in a separate solvent, is provided as a first step. In specific embodiments, the solvent is introduced prior to the first reactant into the reactor.

As a second step, the second reactant is added to the starting mixture.

The Lewis acid can be mixed to the starting mixture at the first step, or added to the starting mixture at the second step, or even part of the Lewis acid can be mixed to the starting mixture at the first step and part of the Lewis acid can be added to the starting mixture at the second step.

This two-step addition of the two reactants enable to obtain 1,4-bis(4-phenoxybenzoyl)benzene with a high purity and a high yield, in an efficient way. In particular, it enables to avoid any prior contacting time between the two reactants as in the methods of the prior art, in which the two reactants have to be first mixed together for several hours and heated before adding the second reactant. It also enables to reduce the production of unwanted by-products such as 4-(4-phenoxybenzoyl)benzoic acid or 4-(4-phenoxybenzoyl)benzoic acid ester.

In some embodiments, terephthaloyl chloride can be the second reactant. In these embodiments the diphenyl ether is therefore the first reactant, which is mixed in the solvent to make the starting mixture. The advantage to add terephthaloyl chloride as the second reactant to the starting mixture is that it enables to reduce the excess amount of the Lewis acid compared to the amount of terephthaloyl chloride introduced into the reactor. It also enables to reduce the excess amount of diphenyl ether compared to the amount of terephthaloyl chloride introduced into the reactor. Terephthaloyl chloride can be added in its solid form or in its liquid form. Alternatively, it can also be added as a suspension

or a colloid, namely as a heterogeneous mixture of solid particles of terephthaloyl chloride in a solvent. The solvent for the suspension/colloid is advantageously the abovementioned reaction solvent. Alternatively, it can also be added as a solution, namely as a homogeneous mixture of liquid terephthaloyl chloride in a solvent. The solvent for the solution is preferably the abovementioned reaction solvent.

In some embodiments, terephthaloyl chloride can be the first reactant. In these embodiments, the diphenyl ether is therefore the second reactant, which is added to the starting mixture. The advantage to use the diphenyl ether as the second reactant is that it has a relatively low fusion temperature (26,9°C) and can therefore be added in its liquid form at a temperature which is close to the ambient temperature. Hence, the diphenyl ether can be added very precisely to the starting mixture. Alternatively, the diphenyl ether can be added to the starting mixture as a solution in a solvent, preferably in the abovementioned solvent. In less preferred embodiments, diphenyl ether is added as a solid, or as a suspension or a colloid in a solvent, preferably in the abovementioned reaction solvent. In addition, the advantage to use the diphenyl ether as the second reactant is that the diphenyl ether is less prone to hydrolysis during the step of addition to the starting mixture than the terephthaloyl chloride.

The Lewis acid can be a solid. In some variations, the Lewis acid can be in a particulate form, such as in the form of granules (having, *e.g.,* a Dv80 of more than 1 mm) or in the form of a powder (having, *e.g.,* a Dv80 of less than 1 mm, and preferably a Dv50 of less than 0.5 mm). Dv80 and Dv50 are respectively the particle sizes at the $80^{th}$ and $50^{th}$ percentiles (in volume) of the cumulative size distribution of the Lewis acid particles. These parameters may be determined by sieving. Alternatively, the Lewis acid can also be added as a suspension or a colloid in a solvent. The solvent for the suspension/colloid is advantageously the abovementioned reaction solvent. Alternatively, it can also be added as a solution in a solvent. The solvent for the solution is preferably the abovementioned reaction solvent.

Lewis acids which may be used include, for example, aluminum trichloride, aluminum tribromide, antimony pentachloride, antimony pentafluoride, indium trichloride, gallium trichloride, boron trichloride, boron trifluoride, zinc chloride, ferric chloride, stannic chloride, titanium tetrachloride, and molybdenum pentachloride. Aluminum trichloride, boron trichloride, aluminum tribromide, titanium tetrachloride, antimony pentachloride, ferric chloride, gallium trichloride, and molybdenum pentachloride are preferred. Aluminum trichloride is particularly preferred.

In some embodiments, the Lewis acid can be mixed to the starting mixture before adding the second reactant to the starting mixture. In particular, when the first reactant is terephthaloyl chloride, the Lewis acid can be mixed to the starting mixture before adding the diphenyl ether to the starting mixture.

In some embodiments, the Lewis acid can be mixed with the second reactant and added together to the starting mixture. In particular, when the second reactant is terephthaloyl chloride, the Lewis acid can be mixed with terephthaloyl chloride and added together to the starting mixture.

In some embodiments, the Lewis acid can be partly mixed to the starting mixture before adding the second reactant to the starting mixture and, partly mixed with the second reactant and added together to the starting mixture.

In some particular embodiments, the weight concentrations and weight ratios of the reactants and of the catalyst are as follows: the concentration of terephthaloyl chloride (relative to the sum of solvent, terephthaloyl chloride, diphenyl ether and Lewis acid introduced into the reactor) is from 3 to 12%, preferably from 5 to 10%;

- the concentration of diphenyl ether (relative to the sum of solvent, terephthaloyl chloride, diphenyl ether and Lewis acid introduced into the reactor) is from 5 to 35%, preferably from 12 to 25%;
- the concentration of Lewis acid (relative to the sum of solvent, terephthaloyl chloride, diphenyl ether and Lewis acid introduced into the reactor) is from 4 to 30%, preferably from 10 to 25%;
- the weight ratio of terephthaloyl chloride to diphenyl ether introduced into the reactor is from 0.2 to 0.6, preferably from 0.3 to 0.5;
- the weight ratio of Lewis acid to terephthaloyl chloride plus diphenyl ether introduced into the reactor is from 0.2 to 0.9, preferably from 0.3 to 0.7.

The addition of the second reactant is preferably performed progressively, over a period of time which can advantageously range from 5 to 600 minutes, preferably from 30 to 300 minutes.

The addition can be performed continuously or with one or more interruptions. If it is performed continuously, it can be conducted at a constant rate of addition. Alternatively, the rate of addition can vary over time.

The starting mixture may preferably be agitated during at least part of the reaction step. Thus, the reactor is preferably provided with an agitation device such as a mechanical stirrer (which may, *e.g.,* comprise one or more impellers) or a recirculation loop with a pump.

Preferably, the starting mixture may be agitated owing to the agitation device during the addition of the second reactant.

Once the addition of the second reactant to the starting mixture is complete, the reaction step may optionally comprise a step of maintaining the starting mixture, preferably under agitation, for a certain time, in order to complete the reaction to the desired degree. Preferably, the mixture is maintained from 0 to 600 min, more preferably from 5 to

180 min.

Once the reaction is completed to the desired degree, the starting mixture becomes designated as a product mixture.

The end of the reaction step corresponds to the beginning of the next step in the method, which is usually a purification step, as described in more detail below.

During the reaction step, 1,4-bis(4-phenoxybenzoyl)benzene-Lewis acid complex is initially produced as a dissolved species, the concentration of which progressively increases.

In preferred embodiments, at some point of time during the reaction step, the concentration of the 1,4-bis(4-phenoxybenzoyl)benzene-Lewis acid complex exceeds the saturation limit of the 1,4-bis(4-phenoxybenzoyl)benzene-Lewis acid complex. In other terms, in these embodiments, the 1,4-bis(4-phenoxybenzoyl)benzene-Lewis acid complex is maintained in a supersaturated state during at least part of the step of reacting terephthaloyl chloride with diphenyl ether.

This saturation limit depends on the conditions of the starting mixture in real time and may vary during the reaction step. It depends on the temperature of the starting mixture, the nature of the solvent and the amount of Lewis-acid.

The 1,4-bis(4-phenoxybenzoyl)benzene-Lewis acid complex is said to be in a supersaturated state when it is dissolved in the starting mixture at a concentration exceeding the saturation limit. The supersaturated state is a metastable state. Therefore, the 1,4-bis(4-phenoxybenzoyl)benzene-Lewis acid complex in a supersaturated state is able to suddenly precipitate if a nucleating agent (*e.g.,* solid particles), such as hydrolyzed terephthaloyl chloride, is present in the starting mixture.

By way of convention, in the present application the concentration of the 1,4-bis(4-phenoxybenzoyl)benzene-Lewis acid complex is indicated by the weight concentration of 1,4-bis(4-phenoxybenzoyl)benzene in the solvent. The same applies to the saturation limit.

The saturation limit can be determined using the following model experiment: predetermined quantities of 1,4-bis(4-phenoxybenzoyl)benzene and Lewis acid are added to a given solvent contained in a glass tube. The contents of the glass tube are agitated by magnetic stirring. The temperature of the mixture within the tube is controlled by placing the tube in a thermofluid. The initial temperature is such that the 1,4-bis(4-phenoxybenzoyl)benzene-Lewis acid complex is initially present as a suspension in the solvent. Then the temperature is gradually increased until the 1,4-bis(4-phenoxybenzoyl)benzene-Lewis acid complex gets fully solubilized in the solvent. At this particular temperature, the saturation limit is equal to the weight concentration of 1,4-bis(4-phenoxybenzoyl)benzene in the mixture. The experiment can be repeated a number of times using another weight concentration of 1,4-bis(4-phenoxybenzoyl)benzene and the same weight ratio of Lewis acid with respect to 1,4-bis(4-phenoxybenzoyl)benzene, so that a temperature / saturation curve can be finally obtained for a given solvent and a given weight ratio of Lewis acid to 1,4-bis(4-phenoxybenzoyl)benzene.

**Fig.1** shows by way of illustration the temperature / saturation curve obtained as described above in ortho-dichlorobenzene as a solvent, with a Lewis acid-to-1,4-bis(4-phenoxybenzoyl)benzene molar ratio of 4.05. If the 1,4-bis(4-phenoxybenzoyl)benzene-Lewis acid complex is dissolved in the solvent at a certain temperature and at a certain weight concentration of 1,4-bis(4-phenoxybenzoyl)benzene and if the corresponding temperature / concentration point is situated above the curve, then the 1,4-bis(4-phenoxybenzoyl)benzene-Lewis acid complex is in the supersaturated state.

It has been found by the present inventors that maintaining the 1,4-bis(4-phenoxybenzoyl)benzene-Lewis acid complex in a supersaturated state during part of the reaction step can result in an increased yield or conversion.

The phenomenon at stake is schematically illustrated in **Fig.2,** which shows the typical evolution of the concentration of the relevant species in the starting mixture during the reaction step. It can be seen that, the 4-(4-phenoxybenzoyl)benzoyl chloride (A) is initially produced in a large amount. Then the concentration of 4-(4-phenoxybenzoyl)benzoyl chloride in the starting mixture reaches a peak and decreases, while the concentration of the desired product (B) increases. The amount of xanthydrol moiety-containing impurities (C) increases over time but remains at a relatively low level.

If the 1,4-bis(4-phenoxybenzoyl)benzene-Lewis acid complex precipitates during the reaction step, it is believed (without wishing to be bound by any theory) that part of the unreacted 4-(4-phenoxybenzoyl)benzoyl chloride is caught in the precipitate and thus substantially prevented from further reacting. In other terms, after the 1,4-bis(4-phenoxybenzoyl)benzene-Lewis acid complex has precipitated, it is believed that the conversion of the remaining 4-(4-phenoxybenzoyl)benzoyl chloride (A) to the desired product (B) is significantly impeded.

Therefore, in preferred embodiments, this precipitation is either prevented or at least postponed during the reaction step, without however keeping the concentration of 1,4-bis(4-phenoxybenzoyl)benzene-Lewis acid complex in the mixture at a low level, which would in turn reduce the overall efficiency and kinetics of the reaction. This is made possible because the 1,4-bis(4-phenoxybenzoyl)benzene-Lewis acid complex remains dissolved in the solvent during at least part of the reaction step even after its concentration has reached the saturation limit.

According to some variations, the 1,4-bis(4-phenoxybenzoyl)benzene-Lewis acid complex precipitates at some point of time during the reaction step (after the concentration of this species has exceeded the saturation limit).

According to alternative variations, the 1,4-bis(4-phenoxybenzoyl)benzene-Lewis acid complex does not precipitate during the reaction step and remains dissolved in the solvent during the entirety of the reaction step.

Preferably, the 1,4-bis(4-phenoxybenzoyl)benzene-Lewis acid complex is dissolved in the solvent during at least 75%, or at least 80%, or at least 85%, or at least 90%, or at least 95%, or at least 98%, or at least 99% of the overall duration of the reaction step.

Preferably, the 1,4-bis(4-phenoxybenzoyl)benzene-Lewis acid complex is still dissolved in the solvent (*i.e.,* has not precipitated), when it is present in an amount of 75 mol.%, (or in other variations 80 mol.%, or 85 mol.%, or 90 mol.%, or 91 mol.%, or 92 mol.%, or 93 mol.%, or 94 mol.%, or 95 mol.%) in the starting mixture, said amount being expressed as the amount of 1,4-bis(4-phenoxybenzoyl)benzene relative to the initial amount of terephthaloyl chloride reactant which was introduced.

Preferably, the 1,4-bis(4-phenoxybenzoyl)benzene-Lewis acid complex is dissolved in the solvent at a 1,4-bis(4-phenoxybenzoyl)benzene weight concentration which is higher by at least 5 %, (or in other variations by at least 10 %, or 15 %, or 20 %, or 25 %, or 30 %, or 35 %, or 40 %) than the saturation limit of the 1,4-bis(4-phenoxybenzoyl)benzene-Lewis acid complex, during part of the reaction step, and for instance during at least 5 minutes, or at least 10 minutes, or at least 15 minutes, or at least 20 minutes, or at least 30 minutes, or at least 40 minutes, or at least 1 hour.

Preferably, the 1,4-bis(4-phenoxybenzoyl)benzene-Lewis acid complex is dissolved in the solvent (without precipitation) at a 1,4-bis(4-phenoxybenzoyl)benzene concentration of more than 5 wt.%, or more than 10 wt.%, or more than 15 wt.%, or more than 20 wt.%, or more than 25 wt.%, or more than 30 wt.%, or more than 35 wt.%, or more than 40 wt.%, or more than 50 wt.%, during part of the reaction step.

In order to maintain the concentration of dissolved 1,4-bis(4-phenoxybenzoyl)benzene-Lewis acid complex in the solvent above the saturation limit, nucleation of the precipitate should be prevented or at least delayed. Several measures can be taken in this respect, in a non-limiting manner, in addition to the quality of the starting materials (already addressed above).

First, the internal walls of the reactor (which are in contact with the starting mixture) should have a low surface roughness. Preferably the surface roughness Ra (as defined in ISO 4287) of the walls should be less than 2 $\mu$m, or less than 1 $\mu$m, or less than 0.8 $\mu$m, or less than 0.5 $\mu$m, or less than 0.2 $\mu$m.

Similarly, it is preferable that all equipment surfaces in contact with the starting mixture during the reaction step, such as the surfaces of an impeller (if any) or the internal surfaces of a recirculation loop, should also have a rugosity of less than 2 $\mu$m, or less than 1 $\mu$m, or less than 0.8 $\mu$m, or less than 0.5 $\mu$m, or less than 0.2 $\mu$m.

Second, the method may comprise a preliminary step of washing the reactor, in order to eliminate all solid residues, such as dust particles or residual product particles from a previous reaction, before providing the starting mixture in the reactor. As an example, this can be achieved by spraying or blasting a liquid or a solid or a suspension on reactor wall, preferably the reaction solvent, or by filling the reactor with a liquid or a suspension under agitation, or by any mechanical action.

Third, the method may comprise a step of eliminating solid particles from the starting mixture, either before the start of the reaction step or during the reaction step, such as by filtration (including, *e.g.,* ultrafiltration).

Fourth, the conditions (in particular intensity) of agitation during the reaction step may be selected in such a manner that the average shear stress (calculated as the product $K_{mo}$ x N where $K_{mo}$ is the Metzner-Otto constant for the impellers used and N is the rotation frequency) for the starting mixture remains below 100 $s^{-1}$, preferably below 50 $s^{-1}$, more preferably below 25 $s^{-1}$, even more preferably below 20 $s^{-1}$ during at least part of the reaction step, and more particularly during the part of the reaction step when the concentration of the dissolved 1,4-bis(4-phenoxybenzoyl)benzene-Lewis acid complex in the solvent is above the saturation limit (such as during the last 5%, or the last 10%, or the last 15%, or the last 20%, or the last 25%, or the last 30%, or the last 35%, or the last 40%, or the last 45%, or the last 50% of the reaction step, relative to the overall duration of the reaction step).

The reaction at stake is exothermic. Preferably, a temperature control system is provided, in order to control the temperature of the starting mixture in the reactor, in particular during and after mixing/adding the Lewis acid to the starting mixture and during and after adding the second reactant to the starting mixture. The temperature control system may in particular comprise a temperature sensor within the reactor and may be configured to cool and/or to heat the starting mixture. Preferably, it is at least configured to cool the starting mixture.

Devices for heating and/or cooling the starting mixture may include a heat exchanger inside the reactor or in a recirculation loop, or a heat exchange fluid circuit in the jacket of the reactor.

When the temperature of the starting mixture increases during the step of adding the second reactant, this can be achieved in three different manners:

- by heating the starting mixture (while preferably also controlling the rate of addition of the second reactant, so as to achieve a targeted increase in temperature);
- by simply controlling the rate of addition of the second reactant so as to achieve a targeted increase in temperature, without providing external cooling or heating; or

- by cooling the starting mixture, while also controlling the rate of addition of the second reactant, so as to achieve a targeted increase in temperature

According to a preferred embodiment, the starting mixture is cooled during and possibly also after the step of adding the second reactant, in order to prevent an excessively large or rapid increase in temperature of the starting mixture as the reactants start reacting with each other.

Preferably, the temperature of the starting mixture is greater than 5°C during at least part of the step of adding the second reactant to the starting mixture. In particular variations of embodiments of the invention, the temperature of the starting mixture is at least 10°C, or at least 15°C, or at least 20 °C, or at least 25°C, or at least 30°C, or at least 35°C, or at least 40°C, or at least 45°C, or at least 50°C, or at least 55°C, or at least 60°C, or at least 65°C, or at least 75°C, or at least 85°C, or at least 95°C, or at least 100°C, or at least 110°C, or about 120°C, during at least part of the step of adding the second reactant to the starting mixture.

It should be noted that, when the temperature is higher, the saturation limit of the 1,4-bis(4-phenoxybenzoyl)benzene-Lewis acid complex is also higher, so that a relatively high temperature during at least part of the reaction step is useful for preventing or delaying the undesired precipitation of the 1,4-bis(4-phenoxybenzoyl)benzene-Lewis acid complex.

Besides, it has surprisingly been found that conducting the reaction step at a relatively high temperature, results in an increase in the yield of 1,4-bis(4-phenoxybenzoyl)benzene, without any significant increase in the level of by-product impurities such as xanthydrol moiety-containing molecules.

On the other hand, the temperature during the step of adding the second reactant to the starting mixture should preferably remain below a certain threshold in order to avoid any significant polymerization of the reactants into a PEKK polymer.

Furthermore, the temperature during the step of adding the second reactant to the starting mixture should remain below the boiling temperature of the solvent.

It is possible to operate the reactor in a pressurized manner so that the temperature in the reactor can reach a higher value without causing the solvent to boil. In this case, the pressure in the reactor can range from 1 bar (atmospheric pressure) to 6 bar, preferably from 1.5 bar to 3 bar.

Alternatively, and preferably, the reaction is performed at atmospheric pressure.

According to some variants of embodiments of the invention, the temperature of the starting mixture does not exceed 180°C, preferably does not exceed 120°C, preferably does not exceed 100 °C, more preferably does not exceed 80°C, even more preferably does not exceed 70°C, during the step of adding the second reactant.

It is believed that it is more critical for the temperature of the starting mixture to be relatively high at the end of the step of adding the second reactant than at the beginning of this step, in order to achieve the advantageous effects of embodiments of the invention.

Accordingly, in some variants of embodiments of the invention, once 90% by weight of the second reactant has been added to the starting mixture (relative to the total weight of the second reactant added to the starting mixture), it is preferred that the temperature of the starting mixture is and remains of at least 5°C, preferably at least 10°C, or at least 15°C, or at least 20°C, or at least 25°C, or at least 30°C, or at least 35°C, or at least 40°C, or at least 45°C, or at least 50°C, or at least 55°C, or at least 60°C, or at least 65°C, or at least 75°C, or at least 85°C, or at least 95°C, or at least 100°C, or at least 110°C, or about 120°C, during the remainder of the step of adding the second reactant to the starting mixture.

In some variants of embodiments of the invention, once 75% by weight of the second reactant has been added to the starting mixture (relative to the total weight of the second reactant added to the starting mixture), it is preferred that the temperature of the starting mixture is and remains of at least 5°C, preferably at least 10°C, or at least 15°C, or at least 20°C, or at least 25°C, or at least 30°C, or at least 35°C, or at least 40°C, or at least 45°C, or at least 50°C, or at least 55°C, or at least 60°C, or at least 65°C, or at least 75°C, or at least 85°C, or at least 95°C, or at least 100°C, or at least 110°C, or about 120°C, during the remainder of the step of adding the second reactant to the starting mixture.

In some variants of embodiments of the invention, once 50% by weight of the second reactant has been added to the starting mixture (relative to the total weight of Lewis acid added to the starting mixture), it is preferred that the temperature of the starting mixture is and remains of at least 5°C, preferably at least 10°C, or at least 15°C, or at least 20°C, or at least 25°C, or at least 30°C, or at least 35°C, or at least 40°C, or at least 45°C, or at least 50°C, or at least 55°C, or at least 60°C, or at least 65°C, or at least 75°C, or at least 85°C, or at least 95°C, or at least 100°C, or at least 110°C, or about 120°C, during the remainder of the step of adding the second reactant to the starting mixture.

In some variants of embodiments of the invention, once 20% by weight of the second reactant has been added to the starting mixture (relative to the total weight of the second of the two reactants added to the starting mixture), it is preferred that the temperature of the starting mixture is and remains of at least 5°C, preferably at least 10°C, or at least 15°C, or at least 20°C, or at least 25°C, or at least 30°C, or at least 35°C, or at least 40°C, or at least 45°C, or at least 50°C, or at least 55°C, or at least 60°C, or at least 65°C, or at least 75°C, or at least 85°C, or at least 95°C, or at least

100°C, or at least 110°C, or about 120°C, during the remainder of the step of adding the second reactant to the starting mixture.

The temperature of the starting mixture can remain constant during the step of adding the second reactant. Alternatively, it can vary during this step.

By *"initial temperature"* is meant the temperature of the starting mixture at the beginning of the step of adding the second reactant, *i.e.,* as the first molecules of Lewis acid are added to the starting mixture.

By *"final temperature"* is meant the temperature of the starting mixture at the end of the step of adding the second reactant, *i.e.,* as the last molecules of Lewis acid are added to the starting mixture.

The initial temperature of the starting mixture may range from, e.g., -30°C to 120°C. In some variations, the initial temperature of the starting mixture is from -30 to -25°C; or from -25 to -20°C; or from -20 to -15°C; or from -15 to -10°C; or from -10 to -5°C; or from -5 to -0°C; or from 0 to 5°C; or from 5 to 10°C; or from 10 to 15°C; or from 15 to 20°C; or from 20 to 25°C; or from 25 to 30°C; or from 30 to 35°C; or from 35 to 40°C; or from 40 to 45°C; or from 45 to 50°C; or from 50 to 55°C; or from 55 to 60°C; or from 60 to 65°C; or from 65 to 70°C; or from 70 to 75°C; or from 75 to 80°C; or from 80 to 85°C; or from 85°C to 90°C; or from 90°C to 100°C; or from 100°C to 110°C; or from 110°C to 120°C. Ranges of from 0 to 80°C, more particularly from 20 to 50°C are preferred.

The final temperature of the starting mixture may range from, *e.g.,* 10°C to 120°C. In some variations, the final temperature of the starting mixture is from 10 to 15°C; or from 15 to 20°C; or from 20 to 25°C; or from 25 to 30°C; or from 30 to 35°C; or from 35 to 40°C; or from 40 to 45°C; or from 45 to 50°C; or from 50 to 55°C; or from 55 to 60°C; or from 60 to 65°C; or from 65 to 70°C; or from 70 to 75°C; or from 75 to 80°C; or from 80 to 85°C; or from 85°C to 90°C; or from 90°C to 100°C; or from 100°C to 110°C; or from 110°C to 120°C. Ranges of from 30 to 80°C, and more particularly from 40 to 70°C, even more particularly from 45 to 60°C are preferred. In some variations, the final temperature is at least 30°C, preferably at least 40°C, more preferably at least 45°C and most preferably at least 50°C.

In some variations, the temperature of the starting mixture decreases during the step of adding the second reactant, *i.e.,* the final temperature is lower than the initial temperature.

In preferred variations, the temperature of the starting mixture increases during the step of adding the second reactant, *i.e.,* the final temperature is greater than the initial temperature.

In some embodiments, the temperature difference $\Delta T$ between the final temperature and the initial temperature is from 1 to 120°C, preferably from 1 to 70°C, preferably from 5 to 60°C, more preferably from 10 to 50°C, and in particular from 20 to 40°C.

In some variations of embodiments of the invention, the increase in temperature is monotonous, *i.e.,* there is no transient decrease in temperature during the entire step of adding the second reactant. On the other hand, transient variations or fluctuations in temperature are possible in some embodiments, especially due to the non-instantaneous nature of the temperature control.

In some variations, the temperature of the starting mixture continuously increases from the initial temperature to the final temperature. Alternatively, the temperature of the starting mixture may comprise one or more increase stages and one more plateau stages during the step of adding the second reactant. In particular, the temperature of the starting mixture may initially increase during a first part of the step of adding the second reactant, from the initial temperature to the final temperature, and then plateau at the final temperature during a second part of the step of adding the second reactant. In this case, the plateau temperature may be set with a precision of, *e.g.,* +/- 5°C, or +/- 2°C, or +/- 1°C.

There is no limitation as to the temperature of the starting mixture during the optional step of maintaining the starting mixture, after the addition of the second reactant. In some variations of embodiments of the invention, the temperature of the mixture is maintained at the final temperature described above. In other variations, it increases or decreases relative to the final temperature.

The method of embodiments of the invention may advantageously comprise one or more steps (after the reaction step) for purifying 1,4-bis(4-phenoxybenzoyl)benzene-Lewis acid complex from the product mixture, and in particular from the reaction solvent, catalyst and unreacted reactants as well as by-products.

The 1,4-bis(4-phenoxybenzoyl)benzene-Lewis acid complex can be put in contact with a decomplexing solvent, the decomplexing solvent being a protic solvent, so as to dissociate the 1,4-bis(4-phenoxybenzoyl)benzene-Lewis acid complex into 1,4-bis(4-phenoxybenzoyl)benzene and into a derivative of Lewis acid which is mostly solubilized in the decomplexing solvent under a form depending on the nature of the decomplexing solvent. For example, the derivative of Lewis acid can be under the form of a ionic salt, metal hydroxides and their counter-ions, metal alkoxides and their counter-ions or any other compound(s) resulting from the reaction of the Lewis acid with the decomplexing solvent.

The decomplexing solvent is advantageously selected so that 1,4-bis(4-phenoxybenzoyl)benzene tends to precipitate. The decomplexing solvent can be an organic solvent, such as methanol, acetic acid, formic acid, ethanol, isopropanol, and benzyl alcohol. Alternatively, the decomplexing solvent can be an aqueous solution such as a solution of hydrochloric acid. Mixtures of the above solvents can also be used, such as an aqueous-organic solvent, e.g., an aqueous solution mixed with methanol.

In specific embodiments, the purification may comprise the steps of:

- mixing the product mixture with a decomplexing solvent, being a protic solvent, so as to provide a product slurry;
- separating 1,4-bis(4-phenoxybenzoyl)benzene from the product slurry, preferably by filtration and washing.

By way of example, methanol may be used as a decomplexing solvent. Alternatively, a solution of hydrochloric acid may also be used as a decomplexing solvent.

The desired product can then be recovered from the product slurry by filtration. If necessary, the desired product can be further purified by methods well-known by the skilled person such as being submitted to washing step(s), and/or recrystallization step(s) and/or distillation step(s) to eliminate or reduce the amount of remaining impurities. In particular, the product can be washed, preferably by a protic solvent such as methanol, and filtrated again, once or several times. Washing can be performed for example by re-slurrying the product in the solvent.

The 1,4-bis(4-phenoxybenzoyl)benzene obtained according to embodiments of the invention can subsequently be used to perform a polymerization reaction so as to make a polyaryletherketone polymer (PAEK). In particular, the 1,4-bis(4-phenoxybenzoyl)benzene obtained according to embodiments of the invention can subsequently be used to perform a polymerization reaction so as to make a polyetherketoneketone polymer (PEKK).

In order to make the PAEK polymer, 1,4-bis(4-phenoxybenzoyl)benzene is reacted with at least one difunctional aromatic acyl chloride.

The difunctional aromatic acyl chloride may be chosen among the list consisting of: terephthaloyl chloride, isophthaloyl chloride, phthaloyl chloride, phosgene, adipoyl dichloride, tetrabromophthaloyl chloride, compounds of the following formula and mixtures thereof:

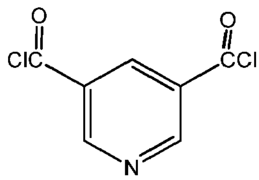
;

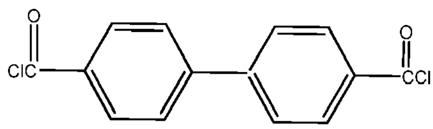
;

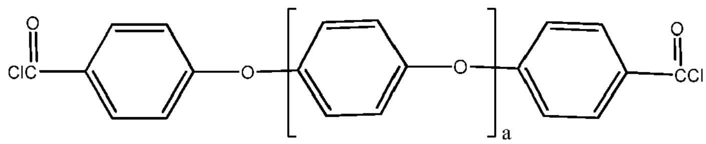
;

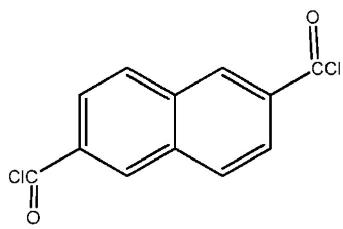
;

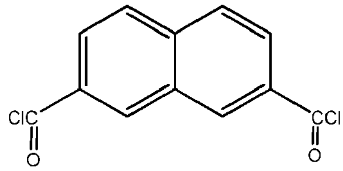
;

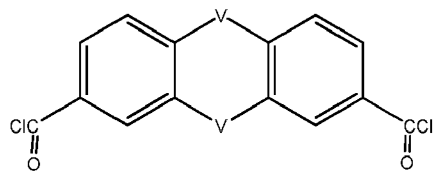

;

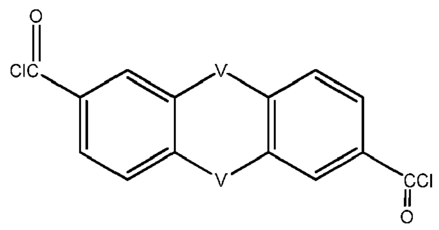

;

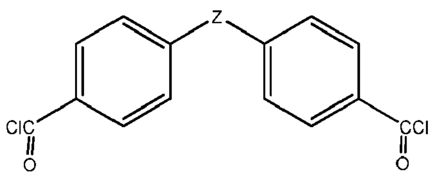

,

wherein:

a is an integer between 0 to 3;
V is chosen among: -O-, -S-, -N=N-, $-(CF_2)_q-$, $-(CH_2)_q-$, or $-C((CH_3)_2)-$;
Z is chosen among -C(O)-, $-SO_2-$, $-C(O)-C_6H_4-C(O)-$, $-O-(CF_2)_q-O-$, -S-, -N=N-, $-(CF_2)_q-$, $-(CH_2)_q-$, or $C-(CH_3)_2-$; and, wherein q is an integer between 1 to 20.

In the embodiment in which the PAEK is PEKK, the difunctional aromatic acyl chloride can be a mixture of phthaloyl chloride, terephthaloyl chloride and isophthaloyl chloride. Preferably, the difunctional aromatic acyl chloride is a mixture of terephthaloyl chloride and isophthaloyl chloride.

The reaction is preferably implemented in a solvent. The solvent is preferably a non-protic solvent, which can in particular be selected from methylene chloride, carbon disulfide, ortho-dichlorobenzene, meta-dichlorobenzene, para-dichlorobenzene, 1,2,4-trichlorobenzene, 1,2,3-trichlorobenzene, ortho-difluorobenzene, 1,2-dichloroethane, 1,1-dichloroethane, 1,1,2,2-tetrachloroethane, tetrachloroethylene, dichloromethane, nitrobenzene and mixtures thereof.

The reaction is preferably implemented in the presence of a Lewis acid as a catalyst.

Lewis acids which may be used include, for example, aluminum trichloride, aluminum tribromide, antimony pentachloride, antimony pentafluoride, indium trichloride, gallium trichloride, boron trichloride, boron trifluoride, zinc chloride, ferric chloride, stannic chloride, titanium tetrachloride, and molybdenum pentachloride. Aluminum trichloride, boron trichloride, aluminum tribromide, titanium tetrachloride, antimony pentachloride, ferric chloride, gallium trichloride, and molybdenum pentachloride are preferred. Aluminum trichloride is particularly preferred.

The polymerization can be implemented in the same reactor as the one used for the production of 1,4-bis(4-phenoxybenzoyl)benzene. But more preferably it is implemented in one or more other reactors.

The polymerization can be carried out at a temperature ranging from, e.g., 20 to 120°C.

The method of making the PAEK polymer advantageously also comprises one or more steps for purifying the PEKK polymer, such as steps of:

- mixing the mixture containing the PEKK polymer with a protic solvent so as to provide a PAEK slurry;
- separating the PAEK polymer from the PAEK slurry, preferably by filtration and washing.

The protic solvent used to make the PAEK slurry may be, e.g., methanol.

The PAEK polymer can then be recovered from the PAEK slurry by filtration. If necessary, the polymer can be washed, preferably by a protic solvent such as methanol, and filtrated again, once or several times. Washing can be performed for example by re-slurrying the polymer in the solvent.

EXAMPLES

The following examples illustrate certain embodiments of the invention without limiting it.

Example 1 (comparative): Addition of $AlCl_3$ on a starting mixture comprising terephthaloyl chloride and diphenyl ether

Terephthaloyl chloride yielding a very clear solution when dissolved at 6.5 wt.% in ortho-dichlorobenzene (turbidity of less than 10 NTU) was used.

In a 250 mL reactor equipped with a magnetic stirrer, with a nitrogen inlet and outlet going to a scrubber system, 157 g of ortho-dichlorobenzene, 17.3 g of terephthaloyl chloride and 42.9 g of diphenyl ether were introduced at 25°C.

After full solubilization, 35.3 g of $AlCl_3$ were slowly added to the reactant mixture during 90 min between 25 to 50°C. After completion of $AlCl_3$ addition, the mixture was kept agitated at 50°C during 3 hours to finish the reaction. Then the mixture was quenched in 3% acidic aqueous solution. After removal of aqueous aluminic phase, a sample of product slurry was analyzed with 1H NMR. Molar ratios for the three following species: 1,4-bis(4-phenoxybenzoyl)benzene (desired product), xanthydrol moiety-containing molecules (by-product) and 4-(4-phenoxybenzoyl)benzoic acid (by-product) was calculated based on the characteristic peaks of the relevant species.

They were expressed as follows for each species:

$$\text{Molar ratio [species]} = [\text{Mol\%[species]} / \sum(\text{Mol\% EKKE} + \text{Mol\% xanthydrol moiety-containing molecules} + \text{Mol\% 4-(4-phenoxybenzoyl)benzoic acid})] \times 100.$$

Example 2 and example 3: Addition of terephthaloyl chloride on a starting mixture comprising AlCl3 and diphenyl ether

In example 2 and example 3, experiments analogous to example 1 were performed but with a different sequence to add the chemicals. Terephthaloyl chloride was added at solid state to the starting mixture, at 25°C, composed of ortho-dichlorobenzene, diphenyl ether and $AlCl_3$.

Terephthaloyl chlorides used in examples 2 and 3 yield suspensions having different turbidities when dissolved at 6.5 wt.% in ortho-dichlorobenzene. In example 2, a very clear solution having a turbidity of less than 10 NTU is observed. In example 3, a cloudy suspension having a turbidity of more than 100 NTU was observed.

Example 4 and example 5: Addition of $AlCl_3$ and terephthaloyl chloride on a starting mixture comprising diphenyl ether

In example 4 and example 5, experiments analogous to example 1 were performed but with a different sequence to add the chemicals.

Terephthaloyl chloride and $AlCl_3$ at solid state were simultaneously added to the starting mixture, at 25°C, composed of ortho-dichlorobenzene and diphenyl ether.

Terephthaloyl chlorides used in examples 4 and 5 are respectively the same as the one used in examples 2 and 3 and yield suspensions having different turbidities when dissolved at 6.5 wt.% in ortho-dichlorobenzene. In example 4, a very clear solution having a turbidity of less than 10 NTU is observed. In example 5, a cloudy suspension having a turbidity of more than 100 NTU was observed.

**Table 1** below summarizes the results obtained for examples 1-5, in terms of ratio of 1,4-bis(4-phenoxybenzoyl)benzene (yield) and ratio of two kinds of by-products: xanthydrol moiety-containing molecules and 4-(4-phenoxybenzoyl)benzoic acid.

**Table 1**

| Example No. | 1 | 2 | 3 | 4 | 5 |
|---|---|---|---|---|---|
| Molar ratio 1,4-bis(4-phenoxybenzoyl)benzene | 95,1 | 95,1 | 94,3 | 95,2 | 95,1 |
| Molar ratio xanthydrol moiety-containing molecules | 4,8 | 4,9 | 5,5 | 4,7 | 4,6 |
| Molar ratio 4-(4-phenoxybenzoyl)benzoic acid | 0,09 | 0,03 | 0,21 | 0,03 | 0,25 |

The 1,4-bis(4-phenoxybenzoyl)benzene obtained in examples 2 and 4, manufactured by using substantially non-hydrolyzed terephthaloyl chloride, has less impurities, in particular less 4-(4-phenoxybenzoyl)benzoic acid than products obtained in examples 3 and 5, manufactured by using terephthaloy chloride which is more hydrolyzed, but still

in the scope of the present invention.

Changing the sequence of addition of the chemicals enable to obtain 1,4-bis(4-phenoxybenzoyl)benzene having slightly less or around the same amount of xanthydrol moiety-containing molecules and 4-(4-phenoxybenzoyl)benzoic acid (examples 2 and 4 in comparison with example 1).

## Claims

1. A method for manufacturing 1,4-bis(4-phenoxybenzoyl)benzene, comprising:

   - providing a solvent, a Lewis acid, a first reactant and a second reactant,
   wherein the first reactant and the second reactant are respectively terephthaloyl chloride and diphenyl ether, or reversely;
   wherein the terephthaloyl chloride is of a purity grade such that, 10 minutes after introducing it at a reference concentration of 6.5 wt.% into ortho-dichlorobenzene containing less than 100 ppm by weight of water, at a temperature of 20°C, a solution is obtained having a turbidity of less than 500 NTU;
   - mixing the first reactant in the solvent to make a starting mixture; and,
   - adding the second reactant to the starting mixture;
   wherein the Lewis acid is mixed, at least partly, to the starting mixture before adding the second reactant to the starting mixture, and/or
   wherein the Lewis acid is mixed, at least partly, with the second reactant and added together to the starting mixture;

   so as to obtain a product mixture comprising a 1,4-bis(4-phenoxybenzoyl)benzene-Lewis acid complex.

2. The method of claim 1, wherein the terephthaloyl chloride is of a purity grade such that:

   - 10 minutes after introducing it at a reference concentration of 6.5 wt.% into ortho-dichlorobenzene containing less than 100 ppm by weight of water, at a temperature of 20°C, a solution is obtained having a turbidity of less than 200 NTU, preferably of less than 100 NTU, more preferably of less than 50 NTU, and most preferably of less than 10 NTU;
   - preferably, 10 hours after introducing it at a reference concentration of 6.5 wt.% into ortho-dichlorobenzene containing less than 100 ppm by weight of water, at a temperature of 20°C, a solution is obtained having a turbidity of less than 500 NTU, preferably of less than 200 NTU, more preferably of less than 100 NTU, even more preferably of less than 50 NTU and most preferably of less than 10 NTU; and
   - more preferably, 24 hours after introducing it at a reference concentration of 6.5 wt.% into ortho-dichlorobenzene containing less than 100 ppm by weight of water, at a temperature of 20°C, a solution is obtained having a turbidity of less than 500 NTU, preferably of less than 200 NTU, more preferably of less than 100 NTU, even more preferably of less than 50 NTU and most preferably of less than 10 NTU.

3. The method of claim 1 or claim 2, wherein the terephthaloyl chloride is kept in a sealed container without contact with ambient air before use.

4. The method of any one of claims 1 to 3, wherein the diphenyl ether and solvent, in combination, contain less than 500 ppm by weight of water, advantageously less than 250 ppm by weight of water, preferably less than 150 ppm by weight of water, more preferably less than 100 ppm by weight of water, and most preferably less than 50 ppm by weight of water.

5. The method of any one of claims 1 to 4, wherein the diphenyl ether and solvent, in combination, contain from 1 to 250 ppm by weight of water, preferably from 2 to 200 ppm by weight of water, more preferably from 3 to 150 ppm by weight of water, even more preferably from 4 to 100 ppm by weight of water and most preferably from 5 to 50 ppm by weight of water.

6. The method of any one of claims 1 to 5, comprising a step of drying the solvent and/or the diphenyl ether before use, preferably by distillation or by contacting with a molecular sieve or with a dehydrating agent.

7. The method of any one of claims 1 to 6, wherein the 1,4-bis(4-phenoxybenzoyl)benzene-Lewis acid complex is dissolved in the solvent at a 1,4-bis(4-phenoxybenzoyl)benzene weight concentration in the solvent which is higher

than the saturation limit of the 1,4-bis(4-phenoxybenzoyl)benzene-Lewis acid complex during at least part of the reaction of the terephthaloyl chloride with the diphenyl ether.

**8.** The method of any one of claims 1 to 7, wherein the 1,4-bis(4-phenoxybenzoyl)benzene-Lewis acid complex is dissolved in the solvent at a 1,4-bis(4-phenoxybenzoyl)benzene weight concentration which is higher by at least 5 %, preferably by at least 10 %, more preferably by at least 20 %, than the saturation limit of the 1,4-bis(4-phenoxybenzoyl)benzene-Lewis acid complex, during part of the reaction of the terephthaloyl chloride with the diphenyl ether.

**9.** The method of any one of claims 1 to 8, wherein the 1,4-bis(4-phenoxybenzoyl)benzene-Lewis acid complex is dissolved in the solvent at a 1,4-bis(4-phenoxybenzoyl)benzene weight concentration of more than 5 wt.%, preferably more than 10 wt.%, more preferably more than 15 wt.%, and most preferably more than 30 wt.%, during part of the reaction of the terephthaloyl chloride with the diphenyl ether.

**10.** The method of any one of claims 1 to 9, wherein the Lewis acid is aluminum trichloride.

**11.** The method of any one of claims 1 to 10, wherein the Lewis acid, the first reactant or the second reactant plays the role of the solvent.

**12.** The method of any one of claims 1 to 10, wherein the solvent is ortho-dichlorobenzene.

**13.** The method of any one of claims 1 to 12, wherein the second reactant is terephthaloyl chloride and,
wherein the Lewis acid is mixed with terephthaloyl chloride and added together to the starting mixture.

**14.** The method of any one of claims 1 to 12, wherein the first reactant is terephthaloyl chloride and,
wherein the Lewis acid is mixed with the starting mixture before adding the diphenyl ether to the starting mixture.

**15.** A method of making a polyaryl ether ketone polymer, comprising:

- manufacturing 1,4-bis(4-phenoxybenzoyl)benzene according to the method of any one of claims 1 to 14;
- reacting said 1,4-bis(4-phenoxybenzoyl)benzene with at least one difunctional aromatic acyl chloride.

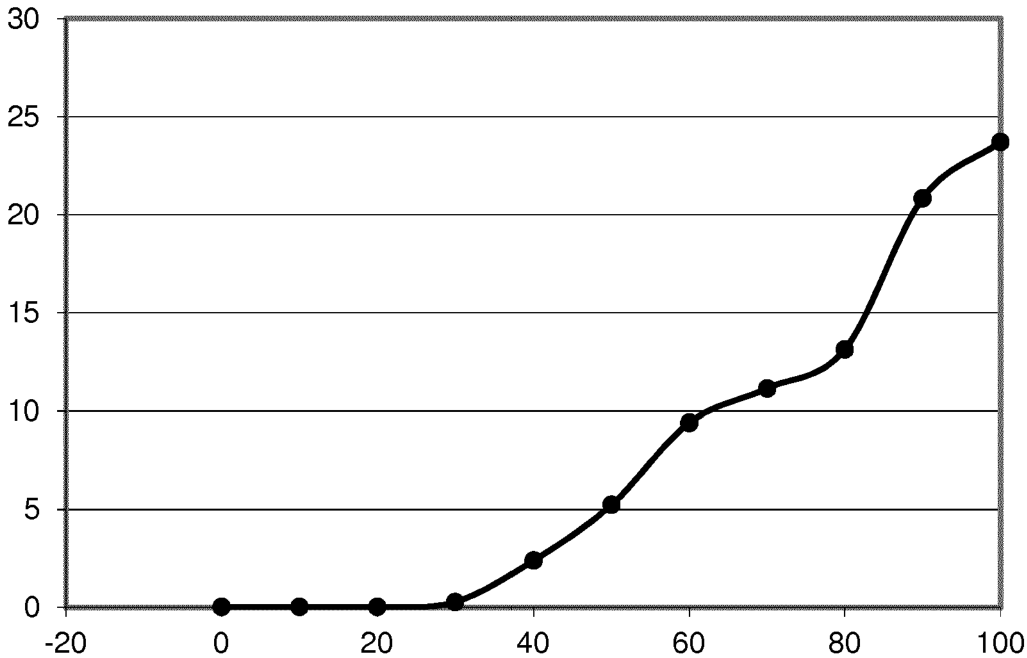
30
25
20
15
10
5
0
-20
0
20
40
60
80
100

Fig. 1

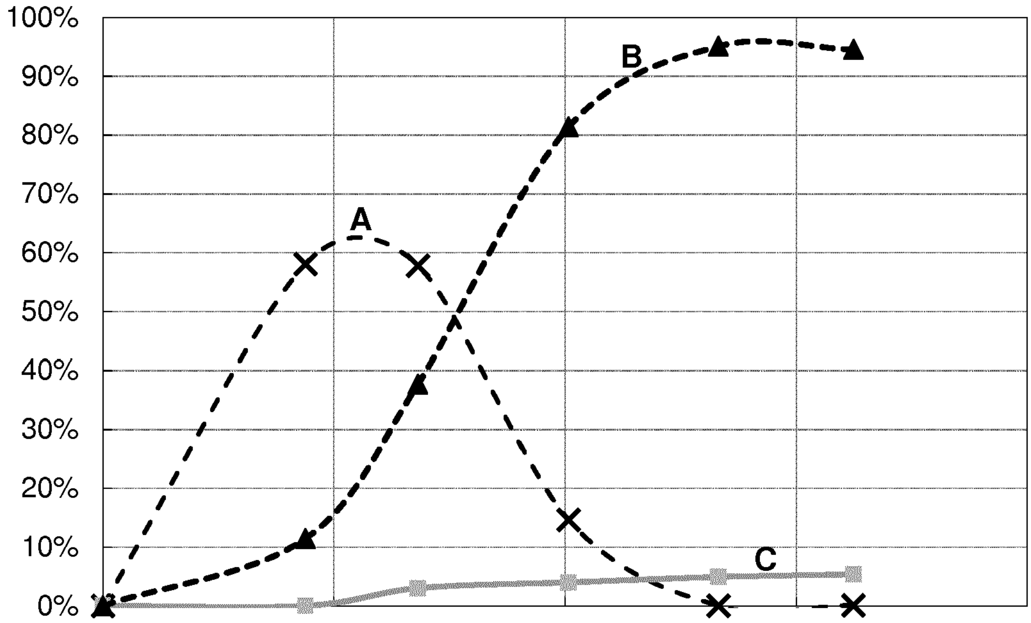
100%
90%
80%
70%
60%
50%
40%
30%
20%
10%
0%
A
B
C

Fig. 2

Europäisches Patentamt
European Patent Office
Office européen des brevets

# EUROPEAN SEARCH REPORT

Application Number
EP 18 30 6472

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 4 826 947 A (JANSONS VIKTORS [US] ET AL) 2 May 1989 (1989-05-02)<br>* example 2 *<br>----- | 1-15 | INV.<br>C07C45/46<br>C07C49/84 |
| X | EP 0 316 133 A2 (DU PONT [US]) 17 May 1989 (1989-05-17)<br>* examples 1, 3-7, 14, 15 *<br>----- | 1-15 | |

TECHNICAL FIELDS SEARCHED (IPC)

C07C

The present search report has been drawn up for all claims

1

| Place of search | Date of completion of the search | Examiner |
|---|---|---|
| The Hague | 10 May 2019 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 30 6472

This annex lists the patent family members relating to the patent documents cited in the above-mentioned European search report. The members are as contained in the European Patent Office EDP file on The European Patent Office is in no way liable for these particulars which are merely given for the purpose of information.

10-05-2019

| Patent document cited in search report | Publication date | Patent family member(s) | Publication date |
|---|---|---|---|
| US 4826947 A | 02-05-1989 | CA 1311077 C | 01-12-1992 |
| | | EP 0298771 A2 | 11-01-1989 |
| | | JP S6438435 A | 08-02-1989 |
| | | US 4826947 A | 02-05-1989 |
| EP 0316133 A2 | 17-05-1989 | CA 1301778 C | 26-05-1992 |
| | | DE 3875587 D1 | 03-12-1992 |
| | | DE 3875587 T2 | 22-04-1993 |
| | | EP 0316133 A2 | 17-05-1989 |
| | | JP H01163149 A | 27-06-1989 |
| | | US 4835319 A | 30-05-1989 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4816556 A
- US 4826947 A
- WO 9523821 A